Europäisches Patentamt

European Patent Office

Office européen des brevets

(19)

(11) EP 0 715 655 B1

(12) EUROPÄISCHE PATENTSCHRIFT

(45) Veröffentlichungstag und Bekanntmachung des Hinweises auf die Patenterteilung:
27.10.1999 Patentblatt 1999/43

(21) Anmeldenummer: 94924197.0

(22) Anmeldetag: 17.08.1994

(51) Int Cl.[6]: **C12Q 1/04**, C12N 1/38

(86) Internationale Anmeldenummer:
PCT/DE94/00953

(87) Internationale Veröffentlichungsnummer:
WO 95/06133 (02.03.1995 Gazette 1995/10)

(54) **VERFAHREN ZUR DETEKTION UND ZÄHLUNG VON MIKROORGANISMEN**

METHOD OF DETECTING AND COUNTING MICROORGANISMS

PROCEDE DE DETECTION ET DE COMPTAGE DE MICRO-ORGANISMES

(84) Benannte Vertragsstaaten:
AT BE CH DE ES FR GB IT LI NL

(30) Priorität: 26.08.1993 DE 4328689

(43) Veröffentlichungstag der Anmeldung:
12.06.1996 Patentblatt 1996/24

(73) Patentinhaber: Beiersdorf Aktiengesellschaft
20245 Hamburg (DE)

(72) Erfinder:
- MEYER, Bianca
  D-22527 Hamburg (DE)
- SAUERMANN, Gerhard
  D-24649 Wiemersdorf (DE)
- TRAUPE, Bernd
  D-22457 Hamburg (DE)
- WOLF, Florian
  D-20251 Hamburg (DE)

(56) Entgegenhaltungen:
- ÄRZTLICHE KOSMETOLOGIE, Bd.13, 1983, KARLSRUHE, DE Seiten 142 - 154 A.A. HARTMANN 'Untersuchungen zu Unterschieden der Keimzahlen der Residentflora benachbarter Hautareale' in der Anmeldung erwähnt
- JOURNAL OF CLINICAL MICROBIOLOGY, Bd.7, Nr.3, März 1978, WASHINGTON, D.C., US Seiten 265 - 272 P. CADY ET AL. 'Electrical Impedance Measurements: Rapid Method for Detecting and Monitoring Microorganisms'
- 'Handbuch Nährböden 'MERCK'', E. MERCK, DARMSTADT, DE siehe Seite 41 - Seite 42 siehe Seite 57 - Seite 58 siehe Seite 50 - Seite 51 siehe Seite 110 siehe Seite 69 - Seite 70 siehe Seite 68
- DIFCO TECHNICAL INFORMATION, Nr.0163, September 1975, DETROIT, MI, US Seiten 6 - 7
- BIOLOGICAL ABSTRACTS, vol. BA75 Philadelphia, PA, US; abstract no. 88933, NIELSEN P A 'ROLE OF REDUCED SULFUR COMPOUNDS IN NUTRITION OF OPIONIBACTERIUM -ACNES.' & J CLIN MICROBIOL 17 (2). 1983. 276-280. CODEN: JCMIDW ISSN: 0095-1137
- BIOLOGICAL ABSTRACTS, vol. BA94 Philadelphia, PA, US; abstract no. 121778, RAN Y-P ET AL 'LOCALIZATION QUANTIFICATION AND BIOCHEMICAL PROPERTIES OF LIPASE OF PITYROSPORUM.' & ZHONGHUA PIFUKE ZAZHI 25 (1). 1992. 8-11, 70. CODEN: CHFTAJ ISSN: 0412-4030
- J. Clin. Microbiol. 17, 2, 1983, 276 - 280
- dmz Lebensmittelindustrie und Milchwirtschaft 31, 1990, 950 - 961
- dmz Lebensmittelindustrie und Milchwirtschaft 32/33, 1991, 992 - 997

**Beschreibung**

Die vorliegende Erfindung betrifft ein Verfahren zur qualitativen Detektion sowie zur quantitativen Zählung von Mikroorganismen, insbesondere solchen Mikroorganismen, die auf der menschlichen Haut angesiedelt sind.

Mikroorganismen werden unterschieden in prokaryontische, also solche ohne eigentlichen Zellkern, und eukaryontische Mikroorganismen, also solche mit einem Zellkern.

Zu den Eukaryonten gehören Algen, Pilze und Protozoen. Zu den Prokaryonten gehören Bakterien. Bakterien wiederum werden klassifiziert in Eubakterien, filamentbildende Bakterien, prosthekate und knospenbildende Bakterien, Actinomyceten, obligat parasitische Bakterien, Spirochäten, Cyanobakterien, Archaebakterien und andere.

Eubakterien werden im wesentlichen unterteilt in Kokken, also kugelförmige Bakterien (z.B. Streptokokken), im wesentlichen stäbchenförmige bzw. gestreckt zylinderförmige nichtsporulierende Bakterien (z.B. coryneforme Bakterien, Propionibakterien, Pseudomonaden, Enterobakterien, sporenbildende Stäbchen (z.B. Bacilli, Clostridien) und gekrümmte Stäbchen (z.B. Spirillen, Vibrionen).

Ferner können die Eubakterien, über die vorab geschilderten Gruppen hinweg, unterteilt werden in Gram-positive und Gram-negative Bakterien, also Bakterien, deren Zellwand nach dem Gram-Test auch nach Waschen mit Alkohol blaugefärbt bleibt oder solche, deren Zellwand durch Alkohol wieder farblos gewaschen wird.

Gram-positive und -negative Bakterien haben, durch die Struktur ihrer Zellwand bedingt, unterschiedliche Eigenschaften, beispielsweise wirkt Penicillin, welches in den Prozeß der Zellwandbildung eingreift, hauptsächlich auf Gram-positive Bakterien (allerdings auch auf einige Gram-negative Bakterien) abtötend.

Mikroorganismen im allgemeinen, aber auch Bakterien im besonderen, sind praktisch allgegenwärtig. Auf der gesunden menschlichen Haut beispielsweise sind hauptsächlich Mycobakterien, Streptokokken, Staphylokokken und Propionibakterien zu finden. Ebenfalls auf der Haut vorkommende coryneforme Bakterien werden neuerdings für die Entstehung unangenehmen Körpergeruches durch die Zersetzung apokrinen Schweißes verantwortlich gemacht.

Pilze, auch Fungi [fungus = lat. Pilz], Mycota [mykes = grch. Pilz] oder Mycobionten genannt, zählen zu den Eucaryonten. Eucaryonten sind Lebewesen, deren Zellen (Eucyten) im Gegensatz zu denen der sogenannten Procaryonten (Procyten) über einen durch Kernhülle und Kernmembran vom restlichen Cytoplasma abgegrenzten Zellkern verfügen. Der Zellkern enthält die Erbinformation in Chromosomen gespeichert.

Zu Vertretern der Mycobionten zählen beispielsweise Hefen (Protoascomycetes), Schimmelpilze (Plectomycetes), Mehltau (Pyrenomycetes), der falsche Mehltau (Phycomycetes) und natürlich die Ständerpilze (Basidiomycetes).

Pilze, auch nicht die Basidiomyceten, sind keine pflanzlichen Organismen, haben aber wie diese eine Zellwand, zellsaftgefüllte Vakuolen und eine mikroskopisch gut sichtbare Plasmaströmung. Sie enthalten keine photosynthetischen Pigmente und sind C-heterotroph. Sie wachsen unter aeroben Bedingungen und gewinnen Energie durch Oxidation organischer Substanzen. Einige Vertreter, beispielsweise Hefen, sind allerdings fakultative Anaerobier und zur Energiegewinnung durch Gärungsprozesse befähigt.

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Dermatomycosen können im wesentlichen in folgende vier Gruppen unterteilt werden: Dermatophytien (z.B. Epidermophytie, Favus, Mikrosporie, Trichophytie), Hefemycosen (z.B. Pityriasis, Candida-Infektionen, Blastomycose, Busse-Buschke-Krankheit, Torulose, Piedra alba, Torulopsidose, Trichosporose), Schimmelmycosen (z.B. Aspergillose, Kephalosporidose, Phycomycose, Skopulariopsidose), Systemmycosen (z.B. Chromomycose, Coccidiomycose, Histoplasmose).

Zu den pathogenen oder fakultativ pathogenen Keimen gehören aus der Gruppe der Hefen Candida-Arten (z.B. Candida albicans) und solche der Familie Pityrosporum. Pityrosporum-Arten, insbesondere Pityrosporum ovale, sind für Hauterkrankungen wie Pityriasis versicolor, Seborrhoe in den Formen Seborrhoea oleosa und Seborrhoea sicca, welche sich vor allem als Seborrhoea capitis (= Kopfschuppen) äußern, seborrhoisches Ekzem und Pityrosporum-Follikulitis verantwortlich zu machen.

Alle Bereiche der menschlichen Haut können von Dermatomycosen befallen werden. Dermatophytien befallen fast ausschließlich Haut, Haare und Nägel. Hefemycosen können auch Schleimhäute und innere Organe befallen, Systemmycosen erstrecken sich regelmäßig auf ganze Organsysteme.

Aber auch bakterielle Infektionen und Superinfektionen der Haut, insbesondere bei immungeschädigten Personen, z.B. Atopikern und Psoriatikern, sowie bei Erkrankungen anderer Genese können erheblichen Leidensdruck verursachen. Unter Superinfektionen ist das übermäßig zahlreiche Auftreten an sich auf der Haut üblicherweise anzutreffender Keime zu verstehen.

Besonders häufig sind die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Besonders unangenehm sind weiterhin Pilzerkrankungen der Finger- und Fußnägelbereiche.

Nicht alle Individuen einer Mikroorganismenpopulation sind lebensfähig. Auf Nähragar Kolonien oder in Nährlösungen Suspensionen bildende Keimen sind lebende, nämlich sich vermehrende Zellen. Zur Bestimmung der Gesamtzellzahl kann man sich einer Zählkammer oder eines elektronischen "Coulter-Counters" bedienen. Zur Bestimmung der Lebendzellzahl können beispielsweise die aus lebenden Zellen hervorgehenden Kolonien ausgezählt werden. Dazu ist es nötig, von verdünnten Zellsuspensionen auszugehen. Derlei Methoden sind dem Fachmann gut vertraut.

Für die klinische Analyse und Diagnostik aber, und diese Begriffe sollen gleichermaßen die dermatologische Analyse der kranken menschlichen Haut im Sinne einer ärztlichen Untersuchung, aber auch die kosmetische Untersuchung an sich gesunder Haut mit kosmetischen, mikrobiell hervorgerufenen Veränderungen umfassen, existieren keine Verfahren, die in kürzester Zeit, unter geringstmöglicher Beanspruchung des Patienten eine zuverlässige qualitative Detektion oder quantitative Auszählung der Keimzahlen ermöglichen. Hier galt es, Abhilfe zu schaffen.

Darüberhinaus hatten die herkömmlichen Zählverfahren des Standes der Technik weitere Nachteile, insbesondere die, daß sie entweder unzuverlässig sind oder erhöhten apparativen, manuellen bzw. zeitlichen Aufwandes bedürfen.

Aufgabe der vorliegenden Erfindung war also, den Mißständen des Standes der Technik Abhilfe zu schaffen. Insbesondere sollten Verfahren zur qualitativen Detektion bzw. quantitativen Auszählung der Keimzahlen zur Verfügung gestellt werden, die auf einfache und schnelle Weise reproduzierbare Ergebnisse liefern.

Weiterhin war es eine Aufgabe der vorliegenden Erfindung, Verfahren zur Verfügung zu stellen, welche ohne allzu großen Aufwand automatisierbar gestaltet werden können.

Eine weitere Aufgabe der vorliegenden Erfindung war es, solche Verfahren zu Verfügung zu stellen, bei welchen einfache Probenahme gewährleistet ist, ohne daß eine Probe vor dem eigentlichen Verfahren noch umständlich und zeitraubend aufgearbeitet werden muß.

Noch eine weitere Aufgabe der vorliegenden Erfindung war, Verfahren zu Verfügung zu stellen, welche möglichst universell für eine möglichst große Vielzahl von Keimen geeignet sind.

Schließlich war es eine Aufgabe der vorliegenden Erfindung, Verfahren zu Verfügung zu stellen, welche speziell für die Mikroorganismenzählung in Bezug auf die Vertreter der menschlichen Hautflora geeignet sind.

Die Lösung all dieser Aufgaben bietet ein
Kosmetisches oder dermatologisches Verfahren zur Detektion und/oder selektiven Quantifizierung einzelner auf menschlicher oder tierischer Haut befindlicher Mikroorganismen bzw. ganzer Mikroorganismengruppen, dadurch gekennzeichnet, daß

(a) nach Entnahme einer Probe von der Mikroflora der menschlichen oder tierischen Haut, welche dadurch erfolgt, daß eine Hautfläche definierter Größe über einen definierten Zeitraum mit einer definierten Menge einer wäßrigen Lösung eines oberflächenaktiven Agens abgespült wird, wobei vorteilhaft diese wäßrige Lösung auf einen pH-Wert abgepuffert ist, der zwischen 5,0 und 8,0 liegt, und wobei die besagte Hautfläche vorteilhaft gleichzeitig unter Ausüben leichten Druckes mit einem Schabewerkzeug, insbesondere eines mit Kunststoff beschichteten Spatels, abgeschabt wird,

(b) diese Probe mit einem Enthemmungsmedium versetzt wird,

(c) die so aufbereitete Probe in ein Kulturmedium gegeben wird, welches günstige Wachstumsbedingungen für eine bestimmte Gruppe von Mikroorganismen aber ungünstige Wachstumsbedingungen für andere Mikroorganismen aufweist, wodurch eine Selektivkultur erzeugt wird und

(d) diese Selektivkultur über einen hinreichend langen Zeitraum bebrütet wird, wodurch lediglich die Gruppe der Mikroorganismen, für welche das Kulturmedium günstige Wachstumsbedingungen aufweist, die Gelegenheit hat, sich zu vermehren,

(e) wobei Stoffwechselprodukte, insbesondere $CO_2$ entstehen, welche sich entweder im Kulturmedium selbst oder einem dafür vorgesehenen Testgefäß, welches ein Indikatormedium enthält, sammeln und

(f) die Konzentration der Stoffwechselprodukte durch die Änderung des Wechselstromwiderstandes des Kulturmediums bzw. des Indikatormediums im Testgefäß ermittelt und, nach entsprechender Eichung, durch rechnerische Methoden mit der Anzahl der Mikroorganismen im Selektivmedium korreliert wird.

Es ist zwar an sich bekannt, Wechselstromwiderstandsmessungen (Impedanzmessungen) bei der Analyse der Stoffwechselvorgänge von Mikroorganismen vorzunehmen. So berichtet beispielweise P.Jaksch ("Grundlagen der Impedanztechnik und Erfahrungen bei der Untersuchung roher und pasteurisierter

Milch" in: dmZ Lebensmittelindustrie und Milchwirtschaft 31, 1991, S. 950 - 960; "Nachweis von Hefen in Joghurt und Frischkäse mittels indirekter Leitfähigkeitsmessung" in: dmZ Lebensmittelindustrie und Milchwirtschaft 32/33, 1991, S. 992 - 996) von Versuchen an Milch und Milchprodukten. Der dermatologisch oder kosmetisch ausgebildete Fachmann konnte jedoch nicht von den an den angegebenen Orten offenbarten Verfahren zu den hiermit als erfindungsgemäß vorgelegten dermatologischen bzw. kosmetischen Analyseverfahren geleitet werden.

Auch werden im Handbuch Nährböden "Merck", E.Merck, Darmstadt, S. 41 -110, ferner in der Schrift J.Clin. Microbiol. 17, 2, 1983, S.276 - 280, ferner in der Schrift Biological Abstracts, Vol. BA94, Abstract no. 121778, ferner in der Schrift DIFCO Technical Information, Nr. 0163, September 1975, Detroit, S. 6-7 sowie ferner in der Schrift J. Clin.Microbiol. 7, 3, 1978, S. 265 - 272 Analysen bzw. Probeentnahmeverfahren beschrieben, die jedoch nicht den Weg zur vorliegenden Erfindung weisen konnten.

Insbesondere hätte der Fachmann annehmen müssen, daß die repräsentative und reproduzierbare Probenentnahme und Probenaufbereitung für Proben der menschlichen oder tierischen Hautmikroflora unmöglich wäre. Der Lebensmitteltechniker, bei dessen Lebensmittelprodukten vergleichsweise große und gut reproduzierbare Probenmengen anfallen, arbeitet eben unter völlig anderen Bedingungen als der Dermatologe oder Kosmetiker, der versuchen muß, repräsentative Mengen eines - ihm zudem noch unbekannten - Mikroorganismus von der Haut zu isolieren.

Wesentlich für die vorliegende Erfindung ist die Verwendung von Wechselstrom, da die Verwendung von Gleichstrom zu elektrolytischer Zersetzung des Testmediums und damit zu einer unkontrollierbaren Veränderung der Meßparameter führen würde.

Der Wechselstromwiderstand eines Objektes setzt sich aus folgenden Einzelerscheinungen zusammen: dem Ohmschen oder reinen Widerstand, dem induktiven Widerstand und dem kapazitiven Widerstand des Objektes. Der Reziprokwert des Ohmschen Widerstandes wird Leitfähigkeit genannt.

Bei der Messung an flüssigen Lösungen spielen induktive Effekte im allgemeinen keine Rolle. Der Wechselstromwiderstand Z, auch Impedanz genannt, errechnet sich aus den Kenngrößen

$G_0 =$ Leitfähigkeit des Mediums
$G_B =$ Effekt der mikrobiellen Stoffwechselaktivität auf die Leitfähigkeit des Mediums
$\Omega =$ Frequenz des Wechselstroms
$C =$ Kapazitiver Widerstand des Mediums

zu:

$$Z = \sqrt{1/(G_0 + G_B)^2 + 1/(\Omega C)^2}$$

Der Term $1/(G_0 + G_B)$ beschreibt den Kehrwert des Ohmschen Widerstandes des Mediums. Die Variable $G_B$ ist abhängig von der Stoffwechselaktivität des zu detektierenden Mikroorganismus, $G_0$ ist konstant. Der den kapazitiven Widerstand betreffende Term $1/(\Omega C)$ kann während einer Messung als konstant angesehen werden, so daß unter den zu wählenden Meßbedingungen die Änderung der Impedanz während einer Messung nur von $G_B$ abhängt.

Dadurch, daß durch die Wahl eines bestimmten erfindungsgemäß verwendbaren Selektivmediums in je einem Arbeitsgang nur jeweils eine einzige Mikroorganismenart bzw. Mikroorganismengattung detektiert wird, deren Stoffwechselaktivität die Impedanzänderung bewirkt, ist es durch geeignete Kalibrierung möglich, einem bestimmten Meßwert für die Impedanz der Mikroorganismenprobe zu einem beliebigen Zeitpunkt direkt ein bestimmten Anzahl des zu detektierenden Mikroorganismus, und zwar eindeutig dieses Mikroorganismus, zuzuordnen.

Die qualitative Detektion wird also durch die Wahl des Selektivmediums erreicht. Die Bestimmung der Anzahl der in einer Probe befindlichen Mikroorganismen einer bestimmten Art wiederum korreliert mit der Konzentration der Stoffwechselprodukte, was erfindungsgemäß durch die Impedanzmessung bestimmt werden kann.

An sich bestehen keine wesentlichen Einschränkungen für die erfindungsgemäß zu verwendenden Impedanzmeßgeräte. Es ergab sich beispielsweise, daß die von der Firma Don Whitley Scientific Limited (Shipley, West Yorkshire, Vereinigtes Königreich) hergestellten und unter der Bezeichnung RABIT (= Rapid Automated Bacterial Impedance Technique) vertriebenen Geräte den Anforderungen bestens gerecht werden. In der Bundesrepublik werden solche Geräte von der Firma Mast Diagnostica verkauft. Aber auch die Geräte anderer Hersteller, beispielsweise der Firmen Malthus oder Sylab sind erfindungsgemäß bestens geeignet.

Grundsätzlich haben sich zwei Meßverfahren für die Impedanz der Mikroorganismenprobe als günstig erwiesen.

Dies sind zum einen die in Fig. 1 dargestellte direkte Meßmethode, bei welcher zwei Elektroden (A, A') direkt in das Selektivmedium (B) eintauchen und die Änderung der Impedanz über den Meßzeitraum verfolgt wird. Fig. 1 zeigt das Prizinzipschaltbild einer Widerstandsmessung, wobei das mit V bezeichnete Meßgerät ein Spannungsmeßgerät, das mit A bezeichnete Gerät ein Strommeßgerät darstellt. Der Gesamtwiderstand Z errechnet sich nach dem Ohmeschen Gesetz, Z = U/I (Spannung/Strom).

**Abb. 1**

Die andere Meßmethode, die in Fig. 2 dargestellt ist, ist insbesondere bei große Mengen $CO_2$ produzierenden Mikroorganismen bevorzugt. Dabei befindet sich in einem Gefäß (C) die Mikroorganismenprobe in Selektivmedium (B). Dieses Gefäß steht über den Luftraum in Verbindung mit einem Indikatormedium (D), welches die von der Mikroorganismenprobe bzw. der sich darin bildenden Mikroorganismenkolonie produzierten gasförmige Stoffwechselprodukte auffängt. Gefäß (C) sowie Indikatormedium (D) befinden sich in einem gasdicht abgeschlossenen Gefäß (E). In dieses Indikatormedium tauchen die Meßelektroden (A, A') ein. Für die Berechnung des Widerstandes Z gilt das zu Figur 1 Gesagte. Als besonders vorteilhaftes Indikatormedium hat sich KOH-Agar erwiesen.

**Fig. 2**

Folgende Meßmethode hat sich als besonders praktikabel erwiesen: Bei einem bestimmten Schwellenwert für die Änderung der Impedanz der Probe, der normalerweise einer Mikroorganismenkonzentration von $10^5$/ml entspricht und etwa -10 µS (Mikrosiemens) bei der indirekten Methode und etwa 5 µS bei der direkten Methode entspricht, wird von der Meßstation ein Signal abgegeben. Das negative Vorzeichen rührt daher, daß sich die Leitfähigkeit des Indikatormediums (KOH-Agar) durch Absorption des produzierten $CO_2$ verringert.

Werden Mikroorganismenkulturen verschiedener Individuenkonzentrationen, die alle unterhalb der Grenzkonzentration liegen, vorgegeben und (gegebenenfalls selektiven) Wachstumsbedingungen unterworfen, so ist die Zeit, nach der die Grenzkonzentration in den einzelnen Proben erreicht und das Signal abgegeben wird, proportional dem Logarithmus der anfänglichen Individuenkonzentration.

Eicht man also den Versuchsaufbau mit Kulturen bekannter Mikroorganismen mit bekannten Individuenkonzentrationen, so kann eine Probe unbekannter Mikroorganismen unbekannter Individuenkonzentration durch die Wahl des Selektivmediums auf die Art des Mikroorganismus geprüft, und durch die Detektion im Impedanzmeßgerät anhand der vorab beschriebenen Meßmethode auf seine Konzentration analysiert werden.

Die käuflichen Impedanzmeßgeräte arbeiten üblicherweise nach dieser Methode.

Es hat sich als vorteilhaft herausgestellt, insbesondere, wenn das Objekt, von welchem die Mikroorganismenprobe entnommen werden soll, die menschliche Haut ist, Methoden der Probeentnahme zu wählen, wie sie in "Untersuchungen zu Unterschieden der Keimzahlen der Residentflora benachbarter Hautareale in Abhängigkeit von der benutzten Hautfloragewinnungsmethode", Ärztliche Kosmetologie 13, S. 142 - 154 (1983) (im Verlag G. Braun, Karlsruhe erschienen), von A.A. Hartmann beschrieben wird.

In dem vorgenannten Artikel werden im wesentlichen folgende Hautfloragewinnungsmethoden beschrieben bzw. zitiert:

| 1.) | Die Detergenswaschmethode | ("DWM") |
| 2.) | Die Cyanoacrylatmethode | ("CyAM") |
| 3.) | Die "water-pick"-Methode | ("S & N") |
| 4.) | Die Wasserspraymethode | ("Thran") |

Es ist insbesondere von Vorteil, den Weg der Detergenswaschmethode einzuschlagen, insbesondere in der nachfolgend beschriebenen Modifikation:

Eine Hautfläche definierter Größe wird über einen definierten Zeitraum mit einer definierten Menge einer wäßrigen Lösung eines oberflächenaktiven Agens abgespült, wobei vorteilhaft diese wäßrige Lösung auf einen pH-Wert abgepuffert ist, der zwischen 5,0 und 8,0 liegt, und wobei die besagte Hautfläche vorteilhaft gleichzeitig unter Ausüben leichten Druckes mit einem Schabewerkzeug, insbesondere eines mit Kunststoff (beispielsweise Teflon) beschichteten Spatels, abgeschabt wird.

Obwohl erfindungsgemäß grundsätzlich alle kosmetisch bzw. dermatologisch geeigneten oberflächenaktive Agentien verwendet werden können, kann es doch gegebenenfalls von Vorteil sein, solche Agentien zu verwenden, welche bereits das Wachstum bestimmter Mikroorganismen eher hemmen und das Wachstum anderer Mikroorganismen nicht oder nur wenig beeinflussen. Das gleiche gilt für die Wahl des pH-Wertes bzw. das Pufferagens selbst: Grundsätzlich können erfindungsgemäße alle kosmetisch bzw. dermatologisch geeigneten Puffer verwendet werden, obwohl es gegebenenfalls von Vorteil sein kann, auch mit der Wahl des Puffers einen gewissen Selektionsdruck auf die Mikroorganismenprobe auszuüben.

Die so gewonnene Probe wird dann mit einem Enthemmungsmedium versetzt. Sie kann sodann mit einem Selektivmedium als Nährboden vereinigt werden, wobei es durchaus möglich ist, zwischen diesen beiden letzteren Ereignissen einen Zeitraum von bis zu sechs Stunden verstreichen zu lassen.

Unter Enthemmungsmedien ist ein Medium zu verstehen, welches eine Reparatur der durch die Abspülung geschädigten Bakterien bewirkt.

Erfindungsgemäß bevorzugte Enthemmungsmedien zeichnen sich durch folgende Zusammensetzungen aus:

| Hirn-Herzextrakt und Pepton | 0 bis 50 g/l |
| D-(*)-Glucose | 0 bis 5 g/l |
| NaCl | 0 bis 20 g/l |
| $Na_2HPO_4 * H_2O$ | 0 bis 10 g/l |
| Tween 80 | 0 bis 50 g/l |
| Lecithin | 0 bis 5 g/l |
| Histidin | 0 bis 5 g/l |

Das Substanzgemisch wird vorteilhaft in 1,0 Liter destillierten Wassers gelöst, autoklaviert und der pH-Wert auf 5,5 bis 8,5 eingestellt.

Das Detektionsverfahren beruht auf dem Umstand, daß das detektierte Stoffwechselprodukt, vorzugsweise $CO_2$, den Widerstand des Testmediums oder Indikatormediums verändert.

Als besonders vorteilhaft verwendbare Verkörperung der vorliegenden Erfindung werden auch Kulturmedien angesehen, welche günstige Wachstumsbedingungen für bestimmte Gruppen von Mikroorganismen, aber ungünstige Wachstumsbedingungen für andere Gruppen von Mikroorganismen aufweisen. Solche Medien werden im Zusammenhang der vorliegenden Erfindung als Selektivmedien bezeichnet.

Erfindungsgemäß bevorzugt verwendbare Selektivmedien werden nachfolgend genauer beschrieben werden. Gleichwohl sind im Rahmen der erfindungsgemäßen Verfahren auch andere, an sich dem Stande der Technik zugehörige Selektivmedien verwendbar.

Es ist im Einzelfalle möglich, daß ein bestimmtes Selektivmedium als Selektivmedium für mehrere an sich unterschiedliche Keime dienen kann. Dies ist aber kein Widerspruch, da es weitere Mechanismen gibt, die Eindeutigkeit des Selektivmediums wieder herzustellen, z.B. wird man in der Mundhöhle vorwiegend Streptokokken finden und kann dennoch ein auf Streptokokken und Enterokokken ansprechendes Selektivmedium verwenden.

Obwohl die erfindungsgemäß verwendbaren Selektivmedien zwar äußerst vorteilhaft als Mittel für die erfindungsgemäßen dermatologischen oder kosmetischen Detektionsverfahren verwendet werden können, ist ihr Anwendungsspektrum jedoch nicht auf solche Verfahren beschränkt.

So können diese Selektivmedien beispielsweise in anderen Detektionsverfahren oder aber auch in Aufzuchtverfahren für Mikroorganismen Anwendung finden.

Die erfindungsgemäß verwendbaren Selektivmedien sind gekennzeichnet durch ein Grundmedium, welches als Nahrungsquelle für Mikroorganismen dient, und darüberhinaus um eine oder mehrere Substanzen, welche das Wachstum bestimmter Mikroorganismen hemmt, andere Mikroorganismen, nämlich die zu detektierenden, unbehelligt läßt.

## Staphylokokken:

Erfindungsgemäß verwendbare Selektivmedien für Staphylokokken bestehen aus 40 - 50 Gewichtsteilen eines üblichen Grundmedium, dem ein Einzelstoff oder ein Stoffgemisch (im Rahmen der vorliegenden Patentanmeldung Selektor bzw. Selektoren genannt), gewählt aus den folgenden Bestandteilen zugegeben werden:

|  | Gew.-Teile |
|---|---|
| Natriumpyruvat | 0,2 bis 20,0 |
| Glycin | 0,05 bis 5,0 |
| KSCN | 0,05 bis 5,0 |
| $NaH_2PO_4$ | 0,05 bis 2,0 |
| $Na_2HPO_4$ | 0,02 bis 2,0 |
| LiCl | 0,1 bis 10,0 |
| Aztreonam | 0 bis 0,01 |
| Linolensäure | 0 bis 50,0 |

Vorteilhaft beträgt die untere Grenze an Selektoren 2 Gewichtsteile auf 50 Gewichtsteile Grundmedium.

Erstaunlicherweise ist es mit Hilfe eines Gehalts an Linolensäure möglich, zwischen Staphylokokken zu differenzieren, insbesondere zwischen Staphylococcus aureus und Staphylococcus epidermidis. Bei Gegenwart von Linolensäure ist Staph. epidermidis zum Wachstum befähigt, im Gegensatz zu Staph. aureus, dessen Wachstum gehemmt wird.

Ein für die Herstellung eines Selektivmediums für Staphylokokken günstiges Grundmedium hat beispielsweise folgende Zusammensetzung:

|  | Gew.-Teile |
|---|---|
| Caseinpepton | 10,0 |
| Fleischextrakt | 5,0 |
| Hefeextrakt | 3,0 |
| Glycerin | 10,0 |
| Agar | 13,0 |

Allerdings ist der Fachmann mit der Zusammenstellung eines das Wachstum von Mikroorganismen förderlichen Grundmediums bestens vertraut, so daß an dieser Stelle nicht weiter darauf eingegangen werden muß. Das gleiche gilt für die anderen im Rahmen dieser Beschreibung offenbarten Grundmedien.

## Propionibacterium spec.:

Erfindungsgemäß verwendbare Selektivmedien für Propionibacterium spec. bestehen aus 35 - 50 Gewichtsteilen eines üblichen Grundmediums, dem ein oder mehrere Selektoren, gewählt aus den folgenden Bestandteilen zugegeben werden:

|  | Gew.-Teile |
|---|---|
| Natriumthioglycolat | 0,05 bis 5,0 |
| NaCl | 0,1 bis 10,0 |
| L-Cystein-HCl | 0,05 bis 5,0 |
| Resazurin | 0,001 bis 0,10 |
| $NaHCO_3$ | 0,05 bis 5,0 |
| Phosphomycin | 0,01 bis 1,0 |

Vorteilhaft beträgt die untere Grenze an Selektoren 0,5 Gewichtsteile auf 50 Gewichtsteile Grundmedium.

Ein für die Herstellung eines Selektivmediums für Propionibacterium spec. günstiges Grundmedium hat beispielsweise folgende Zusammensetzung:

|  | Gew.-Teile |
|---|---|
| Pepton | 15,0 |
| Hefeextrakt | 10,0 |
| Agar | 15,0 |

**Anaerobier:**

Erfindungsgemäß verbwendbare Selektivmedien für Anaerobier bestehen aus 35 - 50 Gewichtsteilen eines üblichen Grundmediums, dem ein oder mehrere Selektoren, gewählt aus den folgenden Bestandteilen zugegeben werden:

|  | Gew.-Teile |
|---|---|
| Natriumthioglycolat | 0,05 bis 5,0 |
| NaCl | 0,1 bis 10,0 |
| L-Cystein-HCl | 0,05 bis 5,0 |
| Resazurin | 0,001 bis 0,10 |
| $NaHCO_3$ | 0,05 bis 5,0 |

Vorteilhaft beträgt die untere Grenze an Selektoren 0,5 Gewichtsteile auf 50 Gewichtsteile Grundmedium.

Ein für die Herstellung eines Selektivmediums für Anaerobier günstiges Grundmedium hat beispielsweise folgende Zusammensetzung:

|  | Gew.-Teile |
|---|---|
| Pepton | 15,0 |
| Hefeextrakt | 10,0 |
| Agar | 15,0 |

**Pityrosporum spec.:**

Erfindungsgemäß verwendbare Selektivmedien für Pityrosporum spec. bestehen aus 40 - 90 Gewichtsteilen eines üblichen Grundmediums, dem ein oder mehrere Selektoren, gewählt aus den folgenden Bestandteilen zugegeben werden:

|  | Gew.-Teile |
|---|---|
| Glycerinmonostearat | 0,05 bis 5,0 |
| Tween 80 | 0,05 bis 5,0 |
| Chloramphenicol | 0,01 bis 1,0 |
| Gentamycin | 0,005 bis 0,5 |

Vorteilhaft beträgt die untere Grenze an Selektoren 0,1 Gewichtsteile auf 50 Gewichtsteile Grundmedium.

Ein für die Herstellung eines Selektivmediums für Pityrosporum spec. günstiges Grundmedium hat beispielsweise folgende Zusammensetzung:

|  | Gew.-Teile |
|---|---|
| Neopeptone | 15,0 |
| Hefeextrakt | 0,1 |
| Agar | 18,0 |
| Olivenöl | 20,0 |

## Hefen, beispielsweise der Gattung Candida:

Erfindungsgemäß verwendbare Selektivmedien für Hefen, beispielsweise der Gattung Candida, bestehen aus 30 - 50 Gewichtsteilen eines üblichen Grundmediums, dem ein oder mehrere Selektoren, gewählt aus den folgenden Bestandteilen zugegeben werden:

|  | Gew.-Teile |
|---|---|
| Wismutsulfit | 0,1 bis 10,0 |
| Neomycin | 0,005 bis 0,5 |

Vorteilhaft beträgt die untere Grenze an Selektoren 0,05 Gewichtsteile auf 50 Gewichtsteile Grundmedium.

Ein für die Herstellung eines Selektivmediums für Hefen günstiges Grundmedium hat beispielsweise folgende Zusammensetzung:

|  | Gew.-Teile |
|---|---|
| Hefeextrakt | 1,0 |
| Agar | 15,0 |
| Glycocoll | 10,0 |
| Glucose | 10,0 |

## Schimmelpilze und Dermatophyten:

Erfindungsgemäß verwendbare Selektivmedien für Schimmelpilze und Dermatophyten bestehen aus 20 - 75 Gewichtsteilen eines üblichen Grundmediums, dem 10 bis 100 Gewichtsteile NaCl zugegeben werden.

## Enterokokken und Streptokokken:

Erfindungsgemäß verwendbare Selektivmedien für Enterokokken, aber auch Streptokokken (beispielsweise Streptococcus mutans) bestehen aus 30 - 60 Gewichtsteilen eines üblichen Grundmediums, dem ein oder mehrere Selektoren, gewählt aus den folgenden Bestandteilen zugegeben werden:

|  | Gew.-Teile |
|---|---|
| Natriumcitrat | 0,5 bis 50,0 |
| Natriumazid | 0,1 bis 10,0 |
| Thalliumacetat | 0,2 bis 20,0 |
| 2,3,5-Triphenyltetrazol (bzw. dessen Säureaddukte) | 0,001 bis 0,10 |

Vorteilhaft beträgt die untere Grenze an Selektoren 5 Gewichtsteile auf 50 Gewichtsteile Grundmedium.

Ein für die Herstellung eines Selektivmediums für Enterokokken, aber auch Streptokokken (beispielsweise Streptococcus mutans) günstiges Grundmedium hat beispielsweise folgende Zusammensetzung:

|  | Gew.-Teile |
|---|---|
| Caseinpepton | 10,0 |

(fortgesetzt)

| | Gew.-Teile |
|---|---|
| Agar | 15,0 |
| Fleischextrakt | 10,0 |
| Glucose | 10,0 |

### Coryneforme Keime und Corynebacterium:

Erfindungsgemäß verwendbare Selektivmedien für coryneforme Keime sowie Corynebacterium bestehen aus 30 - 50 Gewichtsteilen eines üblichen Grundmediums, dem ein oder mehrere Selektoren, gewählt aus den folgenden Bestandteilen zugegeben werden:

| | Gew.-Teile |
|---|---|
| Furazolidon (Aldrich) | 0,5 bis 50,0 |
| Aceton | 0,5 bis 50,0 |
| Tween 80 | 0,1 bis 10,0 |
| Rinderserum | 2,0 bis 200 |
| Phosphomycin (Sigma Chemie) | 5,0 bis 500 |
| Wasser autoklaviert | ad 1000 |

Ein für die Herstellung eines Selektivmediums für coliforme Keime sowie E.coli günstiges Grundmedium hat beispielsweise folgende Zusammensetzung:

| | Gew.-Teile |
|---|---|
| Caso-Agar | 44,00 g |
| Hefeextrakt | 1,10 g |
| Wasser VES | ad 1000,00 ml |

### Coliforme Keime und E.coli :

Erfindungsgemäß verwendbare Selektivmedien für coliforme Keime sowie E.coli bestehen aus 30 - 50 Gewichtsteilen eines üblichen Grundmediums, dem ein oder mehrere Selektoren, gewählt aus den folgenden Bestandteilen zugegeben werden:

| | Gew.-Teile |
|---|---|
| NaCl | 0,1 bis 10,0 |
| Lactose | 0,2 bis 20,0 |
| basisches Fuchsin | 0,02 bis 2,0 |
| $Na_2SO_3$ | 0,05 bis 5,0 |

Vorteilhaft beträgt die untere Grenze an Selektoren 2 Gewichtsteile auf 40 Gewichtsteile Grundmedium.
Ein für die Herstellung eines Selektivmediums für coliforme Keime sowie E.coli günstiges Grundmedium hat beispielsweise folgende Zusammensetzung:

| | Gew.-Teile |
|---|---|
| Pepton aus Fleisch | 10,0 |
| Agar | 20,0 |
| Fleischextrakt | 10,0 |

### Enterobacteriaceen:

Erfindungsgemäß verwendbare Selektivmedien für Enterobacteriaceen, welche das Wachstum grampositiver Bakterien und coliformer Keime vollständig unterdrücken, bestehen aus 30 - 55 Gewichtsteilen eines üblichen Grundmediums, dem ein oder mehrere Selektoren, gewählt aus den folgenden Bestandteilen zugegeben werden:

|  | Gew.-Teile |
|---|---|
| Natriumcitrat | 0,1 bis 10,0 |
| $Na_2S_2O_3$ | 0,1 bis 10,0 |
| Natriumdesoxycholat | 0,1 bis 10,0 |
| Ammoniumeisen-(III)-citrat | 0,05 bis 5,0 |
| Neutralrot | 0,001 bis 0,1 |

Vorteilhaft beträgt die untere Grenze an Selektoren 5 Gewichtsteile auf 40 Gewichtsteile Grundmedium.

Ein für die Herstellung eines Selektivmediums für Enterobacterien günstiges Grundmedium hat beispielsweise folgende Zusammensetzung:

|  | Gew.-Teile |
|---|---|
| Pepton | 5,0 |
| Agar | 13,0 |
| Fleischextrakt | 5,0 |
| Lactose | 10,0 |
| Saccharose | 10,0 |

Erfindungsgemäß besonders vorteilhaft ist, das Verfahren als kosmetisches bzw. dermatologisches Detektionsverfahren anzuwenden. Zu untersuchender Sachverhalt sind dabei Änderungen der Mikroflora der Haut. Die Grenzen zwischen kosmetischer und dermatologischer Untersuchung sind natürlich fließend.

In erster Linie liegt der Schwerpunkt erfindungsgemäßer kosmetischer Detektion in der Bestimmung der Änderung der absoluten bzw. relativen Keimzahlen, weniger der Detektion bestimmter Keime. Allerdings ist auch die Detektion der Gegenwart bestimmter apathogener Keime bei bestimmten kosmetischen Hautveränderungen eine vorteilhafte Ausführungsform der erfindungsgemäßen Verfahren, beispielsweise bei Körpergeruch (Kopf-, Fuß-, Achselgeruch), unreiner Haut, in der Mundhygiene und allen anderen kosmetischen Hautveränderungen, bei welchen Mikroorganismen eine Rolle spielen.

Erfindungsgemäße dermatologische Detektion besteht im wesentlichen in der Bestimmung pathologischer Veränderungen der Mikroflora der Haut. Auch hier kann erfindungsgemäß einesteils vorteilhaft die Gegenwart, aber auch die Anzahl der betreffenden Keime bestimmt werden. Vorteilhaft sind beispielsweise erfindungsgemäße Vefahren zur Detektion folgender dermatologischer Erscheinungsformen:

- atopisches Ekzem
- Psoriasis
- Akne
- seborrhoische Dermatitis
- bakteriell verursachte Cellulitis
- Dermatomycosen
- Superinfektionen der Haut mit pathogenen und/oder apathogenen grampositiven und/oder gramnegativen Mikroorganismen

Die nachfolgenden Beispiele zeigen vorteilhafte Ausführungsformen der erfindungsgemäß zu verwendenden Selektivmedien.

Beispiel 1

Ein besonders vorteilhaftes Selektivmedium für Staphylokokken zeichnet sich durch folgende Zusammensetzung aus:

| Casein-Pepton | 10,00 g |
|---|---|
| Fleischextrakt | 5,00 g |
| Hefeextrakt | 3,00 g |

(fortgesetzt)

| | |
|---|---|
| Glycerin | 10,00 g |
| Na-pyruvat | 10,00 g |
| Glycin | 0,50 g |
| KSCN | 2,25 g |
| $NaH_2PO_4 * H_2O$ | 0,60 g |
| $Na_2HPO_4 * 2 H_2O$ | 0,90 g |
| LiCl | 2,00 g |
| Agar | 13,00 g |

Die Komponenten werden in 1 l Wasser gelöst, autoklaviert und der pH-Wert auf 7,2 eingestellt. Nach Abkühlen auf ca. 50° C werden 10 ml einer 0,45 %-igen Natriumazidlösung zugegeben und die Mischung in Schrägagar-Röhrchen ausgegossen.

Das Medium selektiert auf Staphylokokken. Coryneforme Bakterien zeigen kein Wachstum, und Micrococcus spec. wächst erst nach etwa 40 Stunden, d.i. weit außerhalb der Detektionszeit für Staphylokokken, merklich an.

Beispiel 1a

Ein weiteres besonders vorteilhaftes Selektivmedium für Staphylokokken zeichnet sich durch folgende Zusammensetzung aus:

| | |
|---|---|
| Casein-Pepton | 8,50 g |
| Leberpepton | 2,00 g |
| Hefeextrakt | 2,00 g |
| Lactalbumin | 5,50 g |
| Na-pyruvat | 10,00 g |
| Glycin | 0,50 g |
| $NaH_2PO_4 * H_2O$ | 0,60 g |
| $Na_2HPO_4 * 2 H_2O$ | 0,90 g |
| LiCl | 5,00 g |
| Aztreonam | 0,005 g |
| Agar | 13,00 g |

Die Komponenten werden in 1 l Wasser gelöst, autoklaviert und der pH-Wert auf 7,2 eingestellt. Nach Abkühlen auf ca. 50° C wird die Mischung in Schrägagar-Röhrchen ausgegossen.

Das Medium selektiert auf Staphylokokken.

Beispiel 1b

Ein vorteilhaftes Selektivmedium für Staphylococcus epidermidis gegenüber Staphylococcus aureus zeichnet sich durch folgende Zusammensetzung aus:

| | |
|---|---|
| Casein-Pepton | 8,50 g |
| Leberpepton | 2,00 g |
| Hefeextrakt | 2,00 g |
| Lactalbumin | 5,50 g |
| Na-pyruvat | 10,00 g |
| Glycin | 0,50 g |
| $NaH_2PO_4 * H_2O$ | 0,60 g |
| $Na_2HPO_4 * 2 H_2O$ | 0,90 g |
| LiCl | 5,00 g |
| Aztreonam | 0,005 g |
| Linolensäure | 0,60 g |

(fortgesetzt)

| Agar | 13,00 g |
|------|---------|

Die Komponenten werden in 1 l Wasser gelöst, autoklaviert und der pH-Wert auf 7,2 eingestellt. Nach Abkühlen auf ca. 50° C wird die Mischung in Schrägagar-Röhrchen ausgegossen.

In diesem Medium ist Staphylococcus aureus auch nach 40 Stunden Detektionszeit nicht nachzuweisen.

Beispiel 2

Ein besonders vorteilhaftes Selektivmedium für Propionibacterium spec. zeichnet sich durch folgende Zusammensetzung aus:

| Pepton | 15,0 g |
|--------|--------|
| Hefeextrakt | 10,0 g |
| Natriumthioglycolat | 0,5 g |
| NaCl | 2,5 g |
| L-Cystein-HCl | 0,5 g |
| Resazurin | 0,001 g |
| NaHCO$_3$ | 0,4 g |

Das Substanzgemisch wird in 1,0 l destillierten Wassers gelöst und autoklaviert. Nach Abkühlen auf 50° C werden 160 mg Phosphomycin pro Liter Medium zugesetzt und der pH-Wert auf 7,2 eingestellt.

Beispiel 3

Ein besonders vorteilhaftes Selektivmedium für Anaerobier zeichnet sich durch folgende Zusammensetzung aus:

| Pepton Pepton | 15,0 g 15,0 g |
|---------------|---------------|
| Hefeextrakt | 10,0 g |
| Natriumthioglycolat | 0,5 g |
| NaCl | 2,5 g |
| L-Cystein-HCl | 0,5 g |
| Resazurin | 0,001 g |
| NaHCO$_3$ | 0,4 g |

Das Substanzgemisch wird in 1,0 l destillierten Wassers gelöst und autoklaviert. Der pH-Wert wird auf 7,2 eingestellt. Das eigentliche Detektionsverfahren erfolgt unter anaeroben Bedingungen.

Beispiel 4

Ein besonders vorteilhaftes Selektivmedium für Pityrosporum spec. zeichnet sich durch folgende Zusammensetzung aus:

| Neopeptone | 15,0 g |
|------------|--------|
| Hefeextrakt | 0,1 g |
| Agar | 18,0 g |
| Olivenöl | 20,0 g |
| Glycerinmonostearat | 2,5 g |
| Tween® 80 | 2,0 g |

Das Substanzgemisch wird mit destilliertem Wasser auf 1,0 Liter aufgefüllt, der pH-Wert wird auf 6,0 eingestellt. Sodann wird das Medium autoklaviert. Das noch flüssige Medium wird auf ca. 50° C abkühlen gelassen, 100 mg Chloramphenicol und 50 mg Gentamycin pro Liter werden zugesetzt. Das Medium wird sodann in Schrägagar-

EP 0 715 655 B1

Röhrchen ausgegossen.

Beispiel 5

Ein besonders vorteilhaftes Selektivmedium für Hefen, insbesondere solche der Gattung Candida, zeichnet sich durch folgende Zusammensetzung aus:

| Hefeextrakt | 1,0 g |
|---|---|
| Agar | 15,0 g |
| Glycocoll | 10,0 g |
| Glucose | 10,0 g |
| Wismutsulfit | 7,0 g |

Das Substanzgemisch wird in 1,0 Liter destillierten Wassers gelöst und zum Kochen erhitzt. Nach Abkühlen auf ca. 50° C werden 2,0 mg/l Neomycinsulfat zugegeben und das Medium sodann in Schrägagar-Röhrchen ausgegossen.

Beispiel 6

Ein besonders vorteilhaftes Selektivmedium für Enterokokken, aber auch Streptokokken (beispielsweise Streptococcus mutans) zeichnet sich durch folgende Zusammensetzung aus:

| Caseinpepton | 10,0 g |
|---|---|
| Agar | 15,0 g |
| Fleischextrakt | 10,0 g |
| Glucose | 10,0 g |

Das vorstehende Substanzgemisch für das Grundmedium wird in 1,0 l destilliertem Wasser gelöst und autoklaviert. Nach dem Abkühlen werden zugegeben: 20,0 g Natriumcitrat, 0,02 g Natriumazid, 30 ml einer sterilen wäßrigen 5 %-igen Thallium(III)acetat-Lösung und 10,0 ml einer sterilen wäßrigen 1 %-igen 2,3,5-Triphenyltetrazoliumchlorid-Lösung. Der pH-Wert des so erhaltenen Selektivmediums wird auf 6,2 eingestellt und das Medium in Schrägagarröhrchen ausgegossen.

Das Medium ist besonders gut zum Nachweis von Fäkalkeimen geeignet. Streptokokken, die zur Entstehung von Karies führen, können gleichermaßen nachgewiesen werden, ebenso wie gegen solche Keime (insbesondere Streptococcus mutans) gerichtete antimikrobielle Wirkprinzipien, die in Zahnbehandlungsmitteln enthalten sein können.

Beispiel 7

Ein besonders vorteilhaftes Selektivmedium für coryneforme Keime sowie Corynebacterien zeichnet sich durch folgende Zusammensetzung aus:

| Mischung A | |
|---|---|
| Caso-Agar | 44,00 g |
| Hefeextrakt | 1,10 g |
| Wasser | ad 1000,00 ml |

Die Mischung wird autoklaviert und auf 50° C abkühlen gelassen.

| Mischung B | |
|---|---|
| Furazolidon (Aldrich) | 22,00 mg |
| Aceton | 22,00 ml |
| Tween®80 | 5,50 ml |
| Rinderserum | 100,00 ml |

14

(fortgesetzt)

| Mischung B | |
|---|---|
| Phosphomycin (Sigma Chemie) | 168,00 ml |
| Wasser autoklaviert | 16,80 ml |

Das Furazolidon wird in den 22 ml Aceton, das Phosphomycin wird in den 16,8 ml autoklavierten Wassers gelöst. Die Bestandteile der Mischung B werden dann zusammengegeben und die so entstandene Mischung mit der Mischung A vereinigt.

Beispiel 8

Ein besonders vorteilhaftes Selektivmedium für coliforme Keime sowie E.coli zeichnet sich durch folgende Zusammensetzung aus:

| Pepton aus Fleisch | 10,0 g |
|---|---|
| Agar | 20,0 g |
| Fleischextrakt | 10,0 g |
| NaCl | 5,0 g |
| Lactose | 10,0 g |
| basisches Fuchsin | 0,5 g |
| $Na_2SO_3$ | 2,5 g |

Das vorstehende Substanzgemisch für das Grundmedium wird in 1,0 l destilliertem Wasser gelöst und autoklaviert. Sofern das Medium nach dem Autoklavieren rötlich gefärbt ist, wird noch wenig weiteres Natriumsulfit zugesetzt, bis daß der rötliche Farbton verschwunden ist. Anschließend wird das Medium in Schräg-Agar-Röhrchen ausgegossen.
Nach Verfestigen des Selektivmediums ist dieses umgehend zu verwenden. Nach bereits wenigen Tagen färbt es sich rötlich an, wodurch angezeigt wird, daß es unbrauchbar geworden ist.

Beispiel 9

Ein besonders vorteilhaftes Selektivmedium für Enterobacteriaceen zeichnet sich durch folgende Zusammensetzung aus:

| Pepton | 5,0 g |
|---|---|
| Agar | 13,0 g |
| Fleischextrakt | 5,0 g |
| Lactose | 10,0 g |
| Saccharose | 10,0 g |
| Natriumcitrat | 6,0 g |
| $Na_2S_2O_3$ | 4,0 g |
| Natriumdesoxycholat | 3,0 g |
| Ammoniumeisen- (III) -citrat | 1,0 g |
| Neutralrot | 0,02 g |

Das vorstehende Substanzengemisch wird in 1,0 l destillierten Wassers gelöst und bis zum Kochen erhitzt. Nach Auflösen des Agars wird die Mischung in Schrägagar-Röhrchen ausgegossen und erstarren gelassen.
Das Medium kann auch in der klinischen Diagnostik verwendet werden, z.B. zum Nachweis von Salmonellen, Shigellen, Vibrionen und pathogenen Yersinien.

Beispiel 10

Ein besonders vorteilhaftes Selektivmedium für Schimmelpilze zeichnet sich durch folgende Zusammensetzung aus:

| Malzextrakt | 20,0 g |
|---|---|
| NaCl | 75,0 g |
| Agar | 15,0 g |

Das Substanzgemisch wird in 1,0 Liter destillierten Wassers gelöst, autoklaviert und in Schrägagar-Röhrchen ausgegossen.

Das Medium ist auch zur Kultur von Dermatophyten sowie zum Nachweis antimykotischer Eigenschaften von Testsubstanzen gegenüber solchen Keimen geeignet.

Beispiel 11

| **Enthemmungsmedium** | |
|---|---|
| Hirn-Herzextrakt und Pepton | 27,5 g |
| D-(*)-Glucose | 2,0 g |
| NaCl | 5,0 g |
| $Na_2HPO_4 * H_2O$ | 2,5 g |
| Tween 80 | 30,0 g |
| Lecithin | 3,0 g |
| Histidin | 1,0 g |

Das Substanzgemisch wird in 1,0 Liter destillierten Wassers gelöst, autoklaviert und der pH-Wert auf 7,4 eingestellt.

**Versuch I:**

(1) Kalibrierung des Impedanzsystems für Staphylokokken mit einem Referenzstamm sowie mit Wildisolaten

Als Referenzstamm wurde Staphylococcus epidermidis DSM 20044 verwendet. Es wurde eine aus vierunddreißig verschiedenen Keimzahlen bestehende Eichreihe durch Verdünnung hergestellt und das Impedanzsystem mit Hilfe dieser Eichreihe kalibriert.

Die geringste Keimkonzentration (CFU) betrug $3,3 * 10^1$/ml, entsprechend einer Zeit von 22 Stunden und 24 Minuten bis zum Erreichen des Schwellenwertes von -10 µS, die höchste Konzentration betrug $1,3 * 10^7$/ml, entsprechend einer Zeit von 3 Stunden 12 Minuten bis zum Erreichen des Schwellenwertes von -10 µS.

Es ergab sich eine Eichgerade mit der Beziehung

$$\log (CFU/ml) = mT + c$$

wobei sich

m = -0,255 log (CFU/ml)/hr
c = 7,070 log (CFU/ml)

ergaben. Der Korrelationskoeffizient der linearen Regression betrug -0,942.

Als Wildisolate wurde ein Gemisch von Staphylokokken, die aus der Achselhöhlenregion isoliert worden waren, verwendet. Es wurde eine aus einundfünfzig verschiedenen Keimzahlen bestehende Verdünnungsreihe hergestellt und zur Kalibrierung des Impedanzsystems verwendet.

Es ergab sich eine Eichgerade mit der Beziehung

$$\log (CFU/ml) = mT + c$$

wobei sich

m = -0,204 log (CFU/ml)/hr
c = 7,860 log (CFU/ml)

ergaben. Der Korrelationskoeffizient der linearen Regression betrug -0,917.

(2) Verfahren zur Gewinnung Mikroorganismen von der Haut und zur Detektion und Quantifizierung von Haut-Staphylokokken mittels indirekter Impedanzmessung

Die gesamte Mikroflora eines Bereiches der menschlichen Haut (z.B. der Achsel) wird durch Abspülen einer Fläche von 3,8 cm$^2$ über den Zeitraum von einer Minute mit 1 ml 0,075-molaren Phosphatpuffers (pH = 7,9), enthaltend als oberflächenaktives Agens 0,1 % Triton X-100, unter leichtem Schaben mit einem teflonbeschichteten Metallspatel isoliert.
Die so erhaltene Probe wird mit 500 µl Enthemmungsmedium gemäß Beispiel 11 versetzt.
Je 100 µl der so aufbereiteten Probe werden mit 2,4 ml Selektivmedium gemäß Beispiel 1 versetzt und in der Meßanordnung unter Anwendung der indirekten Detektionsmethode die Keimzahl bestimmt.
Es konnten auf diese Weise bei zehn verschiedenen Probanden ohne Schwierigkeit zwischen 10$^2$ und 10$^7$ Staphylokokken je cm$^2$ Haut nachgewiesen werden.
Als Detektionsgerät wurde ein RABIT der Firma Don Whitley Scientific Ltd. verwendet.

**Versuch II:**

(1) Kalibrierung des Impedanzsystems für coryneforme Bakterien mit einem Referenzstamm

Als Wildstamm wurde Corynebacterium spec. verwendet. Es wurde eine aus einundfünfzig verschiedenen Keimzahlen bestehende Eichreihe durch Verdünnung hergestellt und das Impedanzsystem mit Hilfe dieser Eichreihe kalibriert.
Die geringste Keimkonzentration (CFU) betrug 2,1 * 10$^2$/m1, entsprechend einer Zeit von 51 Stunden und 48 Minuten bis zum Erreichen des Schwellenwertes von -10 µS, die höchste Konzentration betrug 9,8 * 10$^6$/ml, entsprechend einer Zeit von 10 Stunden 24 Minuten bis zum Erreichen des Schwellenwertes von -10 µS.
Es ergab sich eine Eichgerade mit der Beziehung

$$\log (CFU/ml) = mT + c$$

wobei sich

m = -0,085 log (CFU/ml)/hr
c = 7,380 log (CFU/ml)

ergaben. Der Korrelationskoeffizient der linearen Regression betrug -0,883.

(2) Verfahren zur Gewinnung Mikroorganismen von der Haut und zur Detektion und Quantifizierung von coryneformen Bakterien mittels indirekter Impedanzmessung

Die gesamte Mikroflora eines Bereiches der menschlichen Haut (z.B. der Achsel) wird durch Abspülen einer Fläche von 3,8 cm$^2$ über den Zeitraum von einer Minute mit 1 ml 0,075-molaren Phosphatpuffers (pH = 7,9), enthaltend als oberflächenaktives Agens 0,1 % Triton X-100, unter leichtem Schaben mit einem teflonbeschichteten Metallspatel isoliert.
Die so erhaltene Probe wird mit 500 µl Enthemmungsmedium gemäß Beispiel 11 versetzt.
Je 100 µl der so aufbereiteten Probe werden mit 2,4 ml Selektivmedium gemäß Beispiel 7 versetzt und in der Meßanordnung unter Anwendung der indirekten Detektionsmethode die Keimzahl bestimmt.
Es konnten auf diese Weise bei zehn verschiedenen Probanden ohne Schwierigkeit zwischen 10$^2$ und 10$^7$ coryneforme Bakterien bzw. Corynebakterien je cm$^2$ Haut nachgewiesen werden.
Als Detektionsgerät wurde ein RABIT der Firma Don Whitley Scientific Ltd. verwendet.

**Patentansprüche**

1. Kosmetisches oder dermatologisches Verfahren zur Detektion und/oder selektiven Quantifizierung einzelner auf menschlicher oder tierischer Haut befindlicher Mikroorganismen bzw. ganzer Mikroorganismengruppen, dadurch gekennzeichnet, daß

   (a) nach Entnahme einer Probe von der Mikroflora der menschlichen oder tierischen Haut, welche dadurch erfolgt, daß eine Hautfläche definierter Größe über einen definierten Zeitraum mit einer definierten Menge einer wäßrigen Lösung eines oberflächenaktiven Agens abgespült wird, wobei vorteilhaft diese wäßrige Lösung auf einen pH-Wert abgepuffert ist, der zwischen 5,0 und 8,0 liegt, und wobei die besagte Hautfläche vorteilhaft gleichzeitig unter Ausüben leichten Druckes mit einem Schabewerkzeug, insbesondere eines mit Kunststoff beschichteten Spatels, abgeschabt wird,

   (b) diese Probe mit einem Enthemmungsmedium versetzt wird,

   (c) die so aufbereitete Probe in ein Kulturmedium gegeben wird, welches günstige Wachstumsbedingungen für eine bestimmte Gruppe von Mikroorganismen aber ungünstige Wachstumsbedingungen für andere Mikroorganismen aufweist, wodurch eine Selektivkultur erzeugt wird und

   (d) diese Selektivkultur über einen hinreichend langen Zeitraum bebrütet wird, wodurch lediglich die Gruppe der Mikroorganismen, für welche das Kulturmedium günstige Wachstumsbedingungen aufweist, die Gelegenheit hat, sich zu vermehren,

   (e) wobei Stoffwechselprodukte, insbesondere $CO_2$ entstehen, welche sich entweder im Kulturmedium selbst oder einem dafür vorgesehenen Testgefäß, welches ein Indikatormedium enthält, sammeln und

   (f) die Konzentration der Stoffwechselprodukte durch die Änderung des Wechselstromwiderstandes des Kulturmediums bzw. des Indikatormediums im Testgefäß ermittelt und, nach entsprechender Eichung, durch rechnerische Methoden mit der Anzahl der Mikroorganismen im Selektivmedium korreliert wird.

2. Verfahren nach Anspruch 1, dadurch gekennzeichnet, daß Selektivmedien für Staphylokokken verwendet werden, welche 40 - 50 Gewichtsteile eines üblichen Grundmediums sowie weiterhin bis zu 94 Gewichtsteile eines Selektors oder mehrerer Selektoren umfassen, wobei der oder die Selektoren gewählt werden aus der Gruppe Natriumpyruvat, Glycin, KSCN, $NaH_2PO_4$, $Na_2HPO_4$, LiCl.

3. Verfahren nach Anspruch 1, dadurch gekennzeichnet, daß Selektivmedien für Propionibacterium verwendet werden, welche 35 - 50 Gewichtsteile eines üblichen Grundmediums sowie weiterhin bis zu 26 Gewichtsteile eines Selektors oder mehrerer Selektoren umfassen, wobei der oder die Selektoren gewählt werden aus der Gruppe Natriumthioglycolat, NaCl, L-Cystein-HCl, Resazurin, $NaHCO_3$, Phosphomycin.

4. Verfahren nach Anspruch 1, dadurch gekennzeichnet, daß Selektivmedien für Anaerobier verwendet werden, welche 35 - 50 Gewichtsteile eines üblichen Grundmediums sowie weiterhin bis zu 25 Gewichtsteile eines Selektors oder mehrerer Selektoren umfassen, wobei der oder die Selektoren gewählt werden aus der Gruppe Natriumthioglycolat, NaCl, L-Cystein-HCl, Resazurin, $NaHCO_3$.

5. Verfahren nach Anspruch 1, dadurch gekennzeichnet, daß Selektivmedien für Pityrosporum spec. verwendet werden, welche 40 - 90 Gewichtsteile eines üblichen Grundmediums sowie weiterhin bis zu 11 Gewichtsteile eines Selektors oder mehrerer Selektoren umfassen, wobei der oder die Selektoren gewählt werden aus der Gruppe Glycerinmonostearat, Tween 80, Chloramphenicol, Gentamycin.

6. Verfahren nach Anspruch 1, dadurch gekennzeichnet, daß Selektivmedien für Hefen, insbesondere der Gattung Candida, verwendet werden, welche 30 - 50 Gewichtsteile eines üblichen Grundmediums sowie weiterhin bis zu 10 Gewichtsteile eines Selektors oder mehrerer Selektoren umfassen, wobei der oder die Selektoren gewählt werden aus der Gruppe Wismutsulfit, Neomycin.

7. Verfahren nach Anspruch 1, dadurch gekennzeichnet, daß Selektivmedien für Schimmelpilze oder Dermatophyten verwendet werden, welche 20 - 75 Gewichtsteile eines üblichen Grundmediums und 10 bis 100 Gewichtsteile NaCl als Selektor umfassen.

8. Verfahren nach Anspruch 1, dadurch gekennzeichnet, daß Selektivmedien für Enterokokken verwendet werden, welche 30 - 60 Gewichtsteile eines üblichen Grundmediums sowie weiterhin bis zu 80 Gewichtsteile eines Selektors oder mehrerer Selektoren umfassen, wobei der oder die Selektoren gewählt werden aus der Gruppe Natriumcitrat, Natriumazid, Thalliumacetat, 2,3,5-Triphenyltetrazol.

9. Verfahren nach Anspruch 1, dadurch gekennzeichnet, daß Selektivmedien für coliforme Keime sowie Escherichia coli verwendet werden, welche 30 - 60 Gewichtsteile eines üblichen Grundmediums sowie weiterhin bis zu 37 Gewichtsteile eines Selektors oder mehrerer Selektoren umfassen, wobei der oder die Selektoren gewählt werden aus der Gruppe NaCl, Lactose, basisches Fuchsin, $Na_2SO_3$.

10. Verfahren nach Anspruch 1, dadurch gekennzeichnet, daß Selektivmedien für Enterobacterieaceen verwendet werden, welche 30 - 60 Gewichtsteile eines üblichen Grundmediums sowie weiterhin bis zu 35 Gewichtsteile eines Selektors oder mehrerer Selektoren umfassen, wobei der oder die Selektoren gewählt werden aus der Gruppe Natriumcitrat, $Na_2S_2O_3$, Natriumdesoxycholat, Ammoniumeisen-(III)-citrat, Neutralrot.

11. Kosmetisches oder dermatologisches Verfahren nach Anspruch 1 oder 2 zur Detektion folgender dermatologischer Erscheinungsformen: atopisches Ekzem, Psoriasis, Akne, seborrhoische Dermatitis, bakteriell verursachte Cellulitis, Dermatomycosen, Superinfektionen der Haut mit pathogenen und/oder apathogenen grampositiven und/oder gramnegativen Mikroorganismen

## Claims

1. Cosmetic or dermatological method for detecting and/or selectively quantifying individual microorganisms, and/or whole groups of microorganisms, which are present on human or animal skin, characterized in that

   (a) after removing a sample of the microflora of the human or animal skin which is effected by rinsing a skin area of defined size for a defined period of time with a defined quantity of an aqueous solution of a surface-active agent, with this aqueous solution advantageously being buffered to a pH between 5.0 and 8.0, and with the said skin area advantageously being simultaneously scraped, while exerting gentle pressure, with a scraping tool, in particular of a spatula which is coated with a synthetic material,
   (b) this sample is treated with a deinhibiting medium,
   (c) the sample which has been prepared in this way is added to a culture medium which exhibits favourable growth conditions for a defined group of microorganisms but unfavourable growth conditions for other microorganisms, as a result of which a selective culture is produced, and
   (d) this selective culture is incubated over a sufficiently long period of time, such that only the group of microorganisms for which the culture medium exhibits favourable growth conditions has the opportunity to multiply,
   (e) in association with which metabolic products, in particular $CO_2$, are produced which collect either in the culture medium itself or in a test vessel which is provided for the purpose and which contains an indicator medium, and
   (f) the concentration of the metabolic products is ascertained by means of the change in the alternating current (AC) resistance of the culture medium and/or the indicator medium in the test vessel and, after appropriate calibration, is correlated by means of arithmetical methods with the number of microorganisms in the selective medium.

2. Method according to Claim 1, characterized in that selective media for Staphylococci are used which comprise 40-50 parts by weight of a customary base medium and also, in addition, up to 94 parts by weight of a selector or several selectors, with the selector(s) being chosen from the group sodium pyruvate, glycin, KSCN, $NaH_2PO_4$, $Na_2HPO_4$ and LiCl.

3. Method according to Claim 1, characterized in that selective media for Propionibacterium are used which comprise 35-50 parts by weight of a customary base medium and also, in addition, up to 26 parts by weight of a selector or several selectors, with the selector(s) being chosen from the group sodium thioglycolate, NaCl, L-cysteine HCl, resazurin, $NaHCO_3$ and phosphomycin.

4. Method according to Claim 1, characterized in that selective media for anaerobic organisms are used which comprise 35-50 parts by weight of a customary base medium and also, in addition, up to 25 parts by weight of a selector or several selectors, with the selector(s) being chosen from the group sodium thioglycolate, NaCl, L-cysteine HCl,

resazurin and $NaHCO_3$.

5. Method according to Claim 1, characterized in that selective media for Pityrosporum spec. are used which comprise 40-90 parts by weight of a customary base medium and also, in addition, up to 11 parts by weight of a selector or several selectors, with the selector(s) being chosen from the group glycerol monostearate, Tween 80, chloramphenicol and gentamycin.

6. Method according to Claim 1, characterized in that selective media for yeasts, in particular of the genus Candida, are used which comprise 30-50 parts by weight of a customary base medium and also, in addition, up to 10 parts by weight of a selector or several selectors, with the selector(s) being chosen from the group bismuth sulphite and neomycin.

7. Method according to Claim 1, characterized in that selective media for moulds or dermatophytes are used which comprise 20-75 parts by weight of a customary base medium and 10 to 100 parts by weight of NaCl as selector.

8. Method according to Claim 1, characterized in that selective media for enterococci are used which comprise 30-60 parts by weight of a customary base medium and also, in addition, up to 80 parts by weight of a selector or several selectors, with the selector(s) being chosen from the group sodium citrate, sodium azide, thallium acetate and 2,3,5-triphenyltetrazole.

9. Method according to Claim 1, characterized in that selective media for coliform organisms and also Escherichia coli are used which comprise 30-60 parts by weight of a customary base medium and also, in addition, up to 37 parts by weight of a selector or several selectors, with the selector(s) being chosen from the group NaCl, lactose, basic fuchsin and $Na_2SO_3$.

10. Method according to Claim 1, characterized in that selective media for Enterobacterieaceae are used which comprise 30-60 parts by weight of a customary base medium and also, in addition, up to 35 parts by weight of a selector or several selectors, with the selector(s) being chosen from the group sodium citrate, $Na_2S_2O_3$, sodium deoxycholate, ammonium iron(III) citrate and neutral red.

11. Cosmetic or dermatological method according to Claim 1 or 2 for detecting the following dermatological manifestations: atopic eczema, psoriasis, acne, seborrhoeic dermatitis, cellulitis caused by bacteria, dermatomycoses, superinfections of the skin with pathogenic and/or apathogenic Gram-positive and/or Gram-negative microorganisms.

**Revendications**

1. Procédé cosmétique ou dermatologique pour la direction et/ou la détermination quantitative sélective de micro-organismes individuels présents sur la peau humaine ou animale, ou de groupes entiers de micro-organismes, caractérisé en ce que

   (a) après prélèvement d'un échantillon de la microflore de la peau humaine ou animale, qui est effectué par lavage d'une aire de peau de taille définie, pendant un temps défini, avec une quantité définie d'une solution aqueuse d'un agent tensio-actif, cette solution aqueuse étant avantageusement tamponnée à un pH compris entre 5,0 et 8,0, et ladite aire de peau étant avantageusement en même temps raclée avec application d'une légère pression, au moyen d'un instrument de raclage, en particulier d'une spatule revêtue de matière plastique,
   (b) on ajoute un agent de désinhibition à cet échantillon,
   (c) on introduit l'échantillon ainsi préparé dans un milieu de culture qui présente des conditions favorables de croissance pour un groupe déterminé de micro-organismes mais des conditions défavorables de croissance pour d'autres micro-organismes, ce qui permet d'obtenir une culture sélective, et
   (d) cette culture sélective est mise à incuber pendant une durée suffisante pour que seul le groupe de micro-organismes pour lequel le milieu de culture présente les conditions favorables de croissance ait la possibilité de se multiplier,
   (e) ce par quoi il se forme des produits métaboliques, en particulier $CO_2$, qui sont recueillis soit dans le milieu de culture lui-même soit dans un récipient-test prévu à cet effet, qui contient un milieu indicateur, et
   (f) on détermine la concentration des produits métaboliques au moyen de la variation de la résistance en courant alternatif du milieu de culture ou du milieu indicateur dans le récipient-test, et, après étalonnage convenable, on la met en corrélation par des méthodes de calcul avec le nombre des micro-organismes dans le milieu sélectif.

2.  Procédé selon la revendication 1, caractérisé en ce que l'on utilise des milieux sélectifs pour staphylocoques, qui comprennent 40-50 parties en poids d'un milieu de base usuel ainsi qu'en outre jusqu'à 94 parties en poids d'un sélecteur ou de plusieurs sélecteurs, le ou les sélecteurs étant choisis parmi le groupe constitué par le pyruvate de sodium, la glycine, KSCN, $NaH_2PO_4$, $Na_2HPO_4$, LiCl.

3.  Procédé selon la revendication 1, caractérisé en ce que l'on utilise des milieux sélectifs pour *Propionibacterium,* qui comprennent 35-50 parties en poids d'un milieu de base usuel ainsi qu'en outre jusqu'à 26 parties en poids d'un sélecteur ou de plusieurs sélecteurs, le ou les sélecteurs étant choisis parmi le groupe constitué par le thioglycolate de sodium, NaCl, la L-cystéine-HCl, la résazurine, $NaHCO_3$, la phosphomycine.

4.  Procédé selon la revendication 1, caractérisé en ce que l'on utilise des milieux sélectifs pour anaérobies, qui comprennent 35-50 parties en poids d'un milieu de base usuel ainsi qu'en outre jusqu'à 25 parties en poids d'un sélecteur ou de plusieurs sélecteurs, le ou les sélecteurs étant choisis parmi le groupe constitué par le thioglycolate de sodium, NaCl, la L-cystéine-HCl, la résazurine, $NaHCO_3$.

5.  Procédé selon la revendication 1, caractérisé en ce que l'on utilise des milieux sélectifs pour *Pityrosporum spec.,* qui comprennent 40-90 parties en poids d'un milieu de base usuel ainsi qu'en outre jusqu'à 11 parties en poids d'un sélecteur ou de plusieurs sélecteurs, le ou les sélecteurs étant choisis parmi le groupe constitué par le monostéarate de glycérol, le Tween 80, le chloramphénicol, la gentamycine.

6.  Procédé selon la revendication 1, caractérisé en ce que l'on utilise des milieux sélectifs pour levures, en particulier appartenant au genre *Candida,* qui comprennent 30-50 parties en poids d'un milieu de base usuel ainsi qu'en outre jusqu'à 10 parties en poids d'un sélecteur ou de plusieurs sélecteurs, le ou les sélecteurs étant choisis parmi le groupe constitué par le sulfite de bismuth et la néomycine.

7.  Procédé selon la revendication 1, caractérisé en ce que l'on utilise des milieux sélectifs pour moisissures ou dermatophytes, qui comprennent 20-75 parties en poids d'un milieu de base usuel ainsi que 10 à 100 parties en poids de NaCl en tant que sélecteur.

8.  Procédé selon la revendication 1, caractérisé en ce que l'on utilise des milieux sélectifs pour entérocoques, qui comprennent 30-60 parties en poids d'un milieu de base usuel ainsi qu'en outre jusqu'à 80 parties en poids d'un sélecteur ou de plusieurs sélecteurs, le ou les sélecteurs étant choisis parmi le groupe constitué par le citrate de sodium, l'acétate de thallium, le 2,3,5-triphényltétrazole.

9.  Procédé selon la revendication 1 caractérisé en ce que l'on utilise des milieux sélectifs pour germes coliformes ainsi qu'*Escherichia coli,* qui comprennent 30-60 parties en poids d'un milieu de base usuel ainsi qu'en outre jusqu'à 37 parties en poids d'un sélecteur ou de plusieurs sélecteurs, le ou les sélecteurs étant choisis parmi le groupe constitué par NaCl, le lactose, la fuchsine basique, $Na_2SO_3$.

10. Procédé selon la revendication 1, caractérisé en ce que l'on utilise des milieux sélectifs pour *Enterobacteriacae* qui comprennent 30-60 parties en poids d'un milieu de base usuel ainsi qu'en outre jusqu'à 35 parties en poids d'un sélecteur ou de plusieurs sélecteurs, le ou les sélecteurs étant choisis parmi le groupe constitué par le citrate de sodium, $Na_2S_2O_3$, le désoxycholate de sodium, le citrate d'ammonium de fer III, le rouge neutre.

11. Procédé cosmétique ou dermatologique selon la revendication 1 ou 2, pour la détection des manifestations dermatologiques suivantes: l'eczéma atopique, le psoriasis, l'acné, la dermatite séborrhéique, la cellulite d'origine bactérienne, les dermatomycoses, les surinfections de la peau par des micro-organismes à Gram positif et/ou Gram négatif, pathogènes et/ou apathogènes.